# EUROPEAN PATENT APPLICATION

(11) **EP 2 702 937 A1**
(43) Date of publication of application: **05.03.2014**
(21) Application number: 13180188.8
(22) Date of filing: 13.08.2013
(51) Int. Cl.: A61B 5/00, A61B 5/145

(54) **Object information acquiring apparatus**

(30) Priority: 03.09.2012 JP 2012192984
(71) Applicant: Canon Kabushiki Kaisha, Tokyo 146-8501 (JP)
(72) Inventor: Fukutani, Kazuhiko, Tokyo 146-8501 (JP)
(74) Representative: TBK

(57) **Abstract**

An object information acquiring apparatus according to the present invention has: a light source emitting light; a holding member holding an object; a probe receiving an acoustic wave, generated by the object when the light is irradiated on the object via the holding member, and outputting a received signal; and a signal processor acquiring information extracted from the object by using the received signal, wherein a region of the holding member that comes into contact with the object is configured as a non-uniform region that is a region where a normal direction of a tangential plane between the holding member and the object is not constant.

## Description

### BACKGROUND OF THE INVENTION

### Field of the Invention

The present invention relates to an object information acquiring apparatus.

### Description of the Related Art

In recent years, research on light imaging techniques is being actively conducted in the medical field. These techniques involve irradiating light on an object such as a living organism from a light source such as a laser to create an image from information from the object that is obtained based on incident light. One of such light imaging techniques is photoacoustic imaging (PAI).

A photoacoustic imaging apparatus first irradiates pulsed light generated by a light source on an object. Energy of the pulse light that is propagated and diffused inside the object is absorbed by object tissue and instantaneously expands to generate an acoustic wave. In this case, absorption rates of light energy differ according to wavelength among the respective tissues inside the object. Therefore, by controlling wavelength, for example, a stronger acoustic wave can be generated from an imaging object segment such as a tumor as compared to other segments.

Subsequently, the photoacoustic imaging apparatus receives the generated acoustic wave by a probe. By analyzing and processing the received signal, object information such as an optical characteristic distribution and, in particular, an initial sound pressure distribution, a light energy absorption density distribution, or an absorption coefficient distribution inside the object can be obtained. Imaging of the inside of the object can be performed (an image of the inside of the object can be created) by arranging such object information in a format suitable for display.

The object information can also be used for quantitative measurement of a particular substance inside the object such as a level of blood oxygen saturation. In recent years, preclinical research on creating angiograms of small animals using photoacoustic imaging and clinical research on applying the principle of photoacoustic imaging on the diagnosis of breast cancer or the like are being actively conducted.

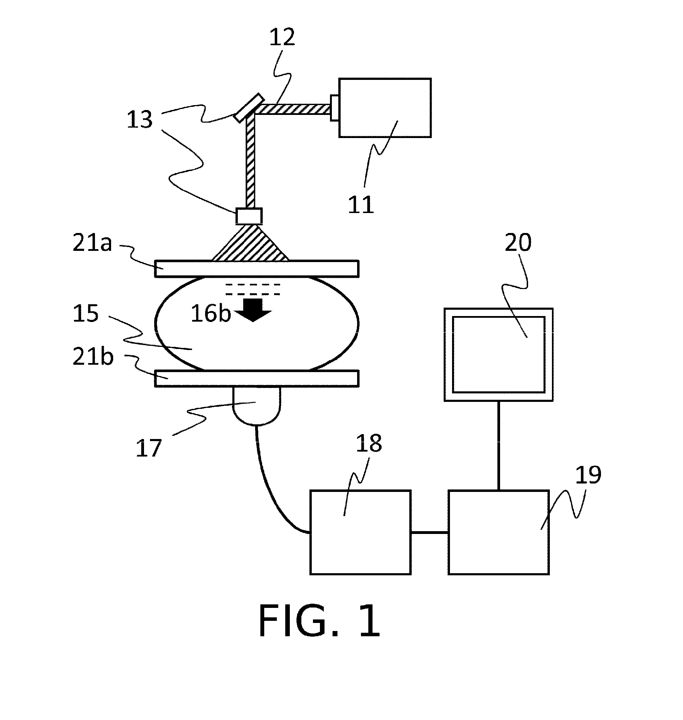

With light imaging including photoacoustic imaging, since light is strongly scattered in a living organism, reaching deep areas with the light is difficult and energy efficiency declines. Therefore, in photoacoustic imaging, when subjecting a large object with a size of several ten mm or more such as a breast to imaging, a method is adopted in which the object is compressed to reduce thickness of the object in a light-irradiating direction in order to increase penetration length of light (Non Patent Literature 1: "Characterization of photoacoustic tomography system with dual illumination", Proceeding SPIE 7899, 7899-91 (2011)). As shown in FIG. 1, with the method described in "Characterization of photoacoustic tomography system with dual illumination", Proceeding SPIE 7899, 7899-91 (2011), the penetration region of light is increased by lightly compressing an object using two flat plates.

Non Patent Literature 1: "Characterization of photoacoustic tomography system with dual illumination", Proceeding SPIE 7899, 7899-91 (2011)

### SUMMARY OF THE INVENTION

However, as shown in FIG. 1, compressing an object 15 using flat plates 21a and 21b results in a planar-shaped object surface. In such a case, the majority of energy of a photoacoustic wave 16b generated from the object surface on a side irradiated by light 12 propagates in a direction perpendicular to a plate surface (a direction indicated by a black arrow). At this point, if an acoustic wave receiving surface of a probe 17 is arranged so as to be parallel to a surface profile of the object, the photoacoustic wave 16b is strongly received by the probe.

When the probe is arranged as shown in FIG. 1, the photoacoustic wave can only be received in a particular direction (this is referred to as a selected area type). An acoustic wave received by a probe in such an arrangement includes a large amount of photoacoustic waves 16b generated from the object surface in addition to those generated inside the object. By calculating an optical characteristic distribution inside the object using a received signal based on such acoustic waves and performing image reconstruction, an image with a large artifact near the object surface on the side irradiated by light is created. As a result, there is a problem in that even if a light absorber such as a blood vessel is present near the object surface on the side irradiated by the light, an image of the light absorber becomes less visible.

The present invention has been made in consideration of the problem described above. The present invention is developed to provide a technique for reducing artifacts when performing photoacoustic imaging while holding an object with a holding member.

The present invention in its first aspect provides an object information acquiring apparatus as specified in claims 1 to 7.

According to the present invention, a technique for reducing artifacts when performing photoacoustic imaging while holding an object with a holding member can be provided.
Further features of the present invention will become apparent from the following description of exemplary embodiments with reference to the attached drawings.

### BRIEF DESCRIPTION OF THE DRAWINGS

FIG. 1 is a diagram showing a configuration of a photoacoustic image-forming apparatus using conventional art;
FIG. 2 is a diagram showing a configuration of a photoacoustic image-forming apparatus using the present invention;
FIGS. 3A to 3F are schematic views showing examples of a holding plate according to the present invention;
FIGS. 4A and 4B are schematic views showing received signals according to the present invention and conventional art;
FIGS. 5A and 5B are schematic views showing reconstructed images according to the present invention and conventional art; and
FIG. 6 is a diagram showing a configuration of a photoacoustic image-forming apparatus according to a second example.

### DESCRIPTION OF THE EMBODIMENTS

Hereinafter, a preferred embodiment of the present invention will be described with reference to the drawings. However, dimensions, materials, shapes, relative arrangements, and the like of components described below are to be modified as appropriate depending on configurations and various conditions of apparatuses to which the invention is applied, and are not intended to limit the scope of the invention to the following description.

The present invention can be favorably applied to object information acquiring apparatuses which perform light imaging and acquire characteristic information from an object. Such object information acquiring apparatuses include apparatuses which irradiate light (electromagnetic waves) on an object and receive acoustic waves generated inside the object, and which acquire object information in the form of image data.

Object information that is acquired by an apparatus utilizing a photoacoustic effect represents a generation source distribution of acoustic waves generated by light irradiation, an initial sound pressure distribution inside an object, or a light energy absorption density distribution, an absorption coefficient distribution, or a concentration distribution of a tissue-forming substance that is derived from the initial sound pressure distribution. Examples of a concentration distribution of a substance include an oxygen saturation distribution and an oxygenated/reduced hemoglobin concentration distribution.

Acoustic waves as described in the present invention are typically ultrasound waves and include elastic waves that are referred to as sound waves, ultrasound waves, or acoustic waves. An acoustic wave generated by a photoacoustic effect is referred to as a photoacoustic wave or a light ultrasound wave. A probe receives acoustic waves generated or reflected inside an object.

Hereinafter, a configuration of a photoacoustic image-forming apparatus that is an example of an object information acquiring apparatus will be described. However, the effect of the present invention is not limited to apparatuses that form images and can also be produced by apparatuses that acquire object information and generate image data. Moreover, as a general rule, the same components will be denoted by the same reference numerals and descriptions thereof will be omitted.

### (Basic configuration)

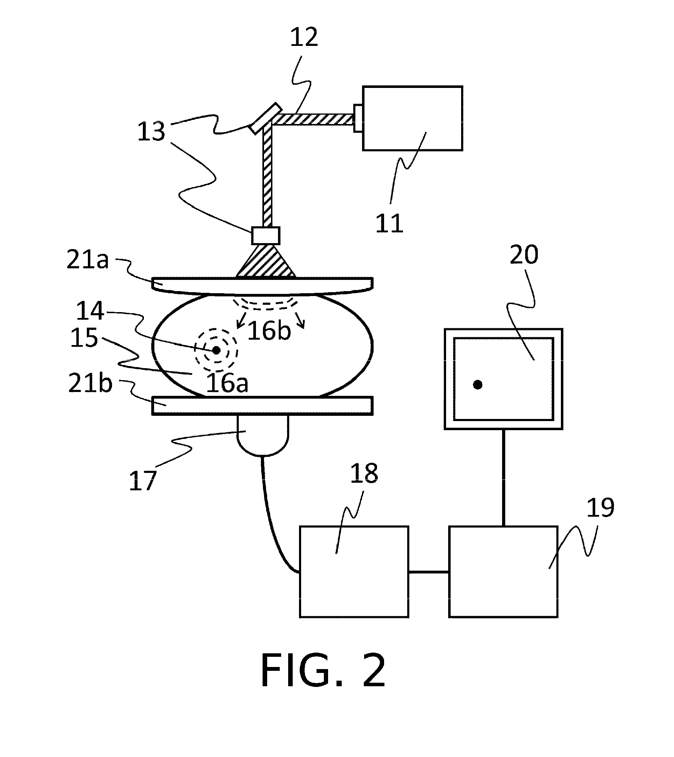

A configuration of a photoacoustic image-forming apparatus according to the present embodiment will now be described with reference to FIG. 2. The photoacoustic image-forming apparatus according to the present embodiment is an apparatus that creates an image from optical characteristic value information representing the inside of an object. Generally, optical characteristic value information represents an initial sound pressure distribution, a light absorption energy density distribution, or an absorption coefficient distribution.

As basic hardware components, the photoacoustic image-forming apparatus according to the present embodiment comprises a light source 11, a holding member 21, a probe 17, and a signal processor 19. The apparatus according to the present embodiment further comprises an optical system 13, a data acquisition system 18, and a display apparatus 20.

Pulsed light 12 emitted by the light source 11 is guided while being processed into a desired light distribution shape by the optical system 13 that is a lens, a mirror, an optical fiber, a diffuser plate, or the like, and is irradiated on an object 15 that is a living organism or the like. In addition, the object 15 is held by the holding member 21. When a part of the energy of light propagated inside the object 15 is absorbed by a light absorber (which eventually becomes a sound source) 14 such as a blood vessel, a photoacoustic wave 16a is generated by a thermal expansion of the light absorber 14. Meanwhile, light energy is also absorbed by a light absorber that exists on the object surface and a photoacoustic wave 16b is generated.

The probe 17 is an acoustic wave receiver. The photoacoustic waves 16a and 16b are received by the probe 17 and converted into an electric signal. The electric signal is amplified or digitally transformed by the data acquisition system 18, and subsequently subjected to predetermined processing at the signal processor 19 and converted into image data (optical characteristic value information data). The display apparatus 20 displays the image data.

Hereinafter, primary components will be described.

### (Holding member 21)

A structure of the holding member 21 that is a feature of the present invention will now be described. The holding member 21 has a function of holding the object or compressing the object to increase a penetration region of light.
A feature of the present embodiment is that a shape of a holding member 21a on a light-irradiating side in a region where the holding member 21a comes into contact with the object is curved as shown in FIG. 2. In other words, a normal direction of a tangential plane of the holding member 21a on the light-irradiating side is not constant in a region where the holding member 21a comes into contact with the object. A region where a normal direction of a tangential plane with an object is not constant may be referred to as a non-uniform region.

Accordingly, energy of a photoacoustic wave generated by the object surface that is in contact with the holding member 21 due to light irradiation propagates in various directions while spreading as shown in FIG. 2 instead of strongly propagating in a direction of a receiving surface of the probe as shown in FIG. 1. As a result, regardless of which direction the receiving surface of the probe faces, received energy of the photoacoustic wave generated by the object surface can be reduced compared to a situation such as that shown in FIG. 1. As a result, compared to the conventional configuration shown in FIG. 1, the configuration according to the present embodiment shown in FIG. 2 significantly reduces reception intensity of a photoacoustic signal at the object surface.

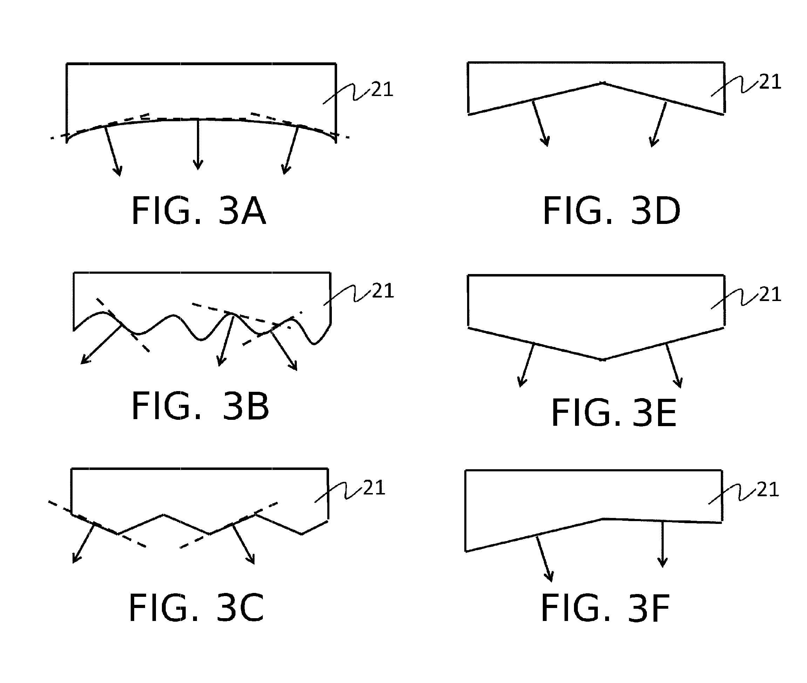

Moreover, FIG. 2 shows an example where a region of the light-irradiating side holding member 21a in contact with the object 15 has a convex shape that protrudes toward the side of the object. However, shapes are not limited thereto. For example, the side in contact with the object may have any of a concave shape such as that shown in FIG. 3A, a wave shape such as that shown in FIG. 3B, or a sawtooth shape such as that shown in FIG. 3C.

Furthermore, the non-uniform region may have an uneven shape without a specific curvature such as the shapes shown in FIGS. 3D, 3E, and 3F. Shapes such as those shown in FIGS. 3D and 3F may also be described as being concave shapes that are depressed relative to the object. In addition, the shape shown in FIG. 3E may also be described as being a convex shape that protrudes toward the object.

In addition, any shape may be adopted as long as normal directions (arrows) of a tangential plane (dashed line) of the holding member 21 are not oriented in a particular direction in a region where the holding member 21 is in contact with the object. In other words, the effect of the present invention can be produced as long as normal directions are not constant.

Moreover, even in a case where two holding members 21 are used, the effect of the present invention can be produced as long as only a surface of the light-irradiating side holding member 21a that is in contact with the object is configured so as to be shaped as described above. On the other hand, a surface of the holding member 21b on the side where light is not irradiated which is in contact with the object may be a planar surface (a normal direction of a tangential plane is always constant).

Moreover, when the holding member 21b is installed between the probe 17 and the object 15 as shown in FIG. 2, desirably, an acoustic impedance of a material of the holding member 21b resembles acoustic impedances of the object and the probe as closely as possible. Accordingly, reflection of acoustic waves at an interface between the probe 17 and the object 15 can be reduced. In addition, when irradiating light on the object surface via a holding member, the holding member is desirably made of an optically-transparent material in order to allow light to pass through.

When the object is a living organism, examples of members that satisfy such acoustic and optical conditions include polymethylpentene, a water pad, and a gel pad which are transparent and which have acoustic impedances that are close to that of a living organism. Moreover, if acoustic impedance need not be considered, any optically-transparent material which allows light to pass through may suffice. Typically, using a plastic plate made of acryl or the like or using a glass plate and the like enables light to be irradiated via the holding member.

The holding member 21 may be of any size as long as the object can be held. However, in consideration of holding, the holding member 21 is favorably larger than the object. For example, when the object is a breast, a size of around 20 x 15 cm is typically used. Although any thickness may be adopted as long as the object can be held, the holding member is favorably as thin as possible in order to minimize multiple reflection of acoustic waves by the holding member. Typically, the thickness is around 1 to 30 mm.

### (Light source 11)

The light source 11 irradiates light on the object 14 and generates a photoacoustic wave 16. When the object 15 is a living organism, the light source 11 irradiates light with a specific wavelength that is absorbed by a specific component among components that constitute the object. Moreover, if the object is a living organism such as a breast, possible light-absorbing components include hemoglobin, fat, water, melanin, and collagen. In addition, when the objective of photoacoustic imaging is to perform blood vessel (blood) imaging, oxygenated hemoglobin or reduced hemoglobin that is included in large amounts in red blood cells in the blood is a measurement object.

When the object is a living organism, a penetration region of light in the living organism is desirably increased as described above. Therefore, normally, a wavelength region in a range of 500 to 1200 nm that is less absorbed by the living organism is used. On the other hand, a wavelength region equal to or less than 500 nm or equal to or more than 1200 nm may be used when light need not be propagated deeply.

The light source 11 may be integrally provided with the photoacoustic imaging apparatus according to the present embodiment, or the light source may be separated and provided as a separate body. As the light source, a pulsed light source capable of generating pulsed light in the order of several nanoseconds to several hundred nanoseconds as irradiated light is favorable. Specifically, a pulse width of around 10 nanoseconds is used in order to generate photoacoustic waves in an efficient manner.

While a laser is capable of producing a large output and is therefore favorably used as the light source, a light-emitting diode or the like can be used instead of a laser. Various lasers can be used including a solid-state laser, a gas laser, a fiber laser, a dye laser, and a semiconductor laser. Moreover, typically, a solid-state laser capable of producing large-output pulsed light such as a YAG laser is used. Timing, waveform, intensity, and the like of irradiation are controlled by a light source controller (not shown). In addition, when measuring an absorption spectrum of a light absorber in a living organism, a light source capable of emitting a plurality of wavelengths is favorable. Examples of such light sources include a Ti:Sa laser, an alexandrite laser, and a dye laser.

### (Probe 17)

The probe 17 is a receiver which receives the photoacoustic wave 16 and converts the photoacoustic wave 16 into an electric signal that is an analog signal. As the probe 17, for example, a transducer using a piezoelectric phenomenon, a transducer using resonance of light, or a transducer using a variation in capacity may be used. Any probe may be used as long as acoustic wave signals can be detected. As the probe 17, a probe having a plurality of receiving elements arranged one-dimensionally or two-dimensionally can be preferably used. Using such multidimensionally-arranged elements enables acoustic waves to be simultaneously received at a plurality of locations, thereby reducing reception time and reducing the effect of vibration of the object or the like.

Accurate imaging of characteristics of a light absorber inside an object requires that photoacoustic waves be received while completely surrounding (over 360 degrees or 4π steradians) the object.

However, when the object is human tissue such as the breast, a probe cannot realistically be provided so as to surround the object. Even in the present embodiment, the probe 17 is only capable of receiving photoacoustic waves generated inside the object from a particular direction. The problem addressed by the present invention occurs in such circumstances. The probe according to the present invention is assumed to be only capable of receiving photoacoustic waves propagated in a particular direction or, in other words, photoacoustic waves propagated via the holding member in contact as shown in FIG. 2. Such an acoustic wave reception system is referred to as a selected area system. An image of an optical characteristic distribution of a light absorber inside the object created by image reconstruction based on data received according to this method is known to include artifacts generated in the image.

### (Signal processor 19)

The signal processor 19 acquires image data inside the object through processing for reducing noise in a received signal or through image reconstruction. Typically, a work station or the like is used as the signal processor 19, and an image reconstruction process or the like is performed by pre-programmed software. For example, software used by the work station is constituted by two modules, namely, a signal processing module which performs a noise reduction process on a received signal and an image reconstruction module which performs image reconstruction using a signal processed by the signal processing module. The image reconstruction module forms image data through image reconstruction.

As an image reconstruction algorithm, for example, image reconstruction methods such as back projection, Fourier transform, and inverse problem analysis by repetitive processing (a model-based method) may be used. A graphics processing unit (GPU) mounted to a work station that is the signal processor 19 is favorably used to reduce image reconstruction time.

In addition, the data acquisition system 18 and the signal processor 19 may be integrated with each other. In this case, image data of the object can be generated by a hardware process instead of a software process performed by a work station or the like.

Hereinafter, non-primary components that are used in an ordinary photoacoustic image-forming apparatus will be described with reference to FIG. 2.

### (Optical system 13)

Light 12 irradiated from the light source 11 is typically guided to the object while being processed into a desired light distribution shape by an optical part such as a lens or a mirror. Alternatively, the light 12 can be propagated using an optical waveguide such as an optical fiber. For example, the optical system 13 is a mirror that reflects light, a lens that collects, expands, or changes the shape of light, or a diffuser plate that diffuses light. Any optical part may be used as long as the light 12 emitted from the light source is irradiated on the object 15 in a desired shape. Moreover, from the perspectives of safety to the object and securing a wider diagnostic region, light is favorably spread over a certain area rather than collected by a lens.

### (Object 15 and light absorber 14)

While the object 15 and the light absorber 14 do not constitute parts of the photoacoustic image-forming apparatus according to the present invention, a description will be given below. A main object of the photoacoustic image-forming apparatus according to the present invention is to diagnose malignant tumors, vascular diseases, or the like in a human or animal, follow up chemical treatment, and the like. Therefore, as the object 15, specific objects such as diagnosis subject segments including the breasts, fingers, hands, and arms of a human or an animal are assumed. The light absorber 14 inside the object exhibits a relatively high absorption coefficient inside the object. For example, if a human body is the measurement subject, malignant tumors which include any of a large amount of oxygenated hemoglobin or reduced hemoglobin, blood vessels including a large amount of oxygenated hemoglobin or reduced hemoglobin, and new blood vessels correspond to the light absorber 14. In addition, examples of a light absorber on the object surface include melanin that is present near the skin surface.

### (Signal collector 18)

The photoacoustic image-forming apparatus according to the present embodiment favorably comprises the data acquisition system 18 which amplifies an electric signal obtained from the probe 17 and which converts the electric signal from an analog signal to a digital signal. The data acquisition system 18 is typically constituted by an amplifier, an A/D converter, a field programmable gate array (FPGA) chip, and the like. When a plurality of received signals is obtained from the probe, desirably, the plurality of signals is simultaneously processed. Accordingly, the time required to form an image can be reduced. Moreover, a "received signal" as used in the present specification is a concept which includes both analog signals acquired from the probe 17 and subsequently AD-converted digital signals. In addition, a received signal is also referred to as a "photoacoustic signal".

### (Display apparatus 20)

The display apparatus 20 is an apparatus for displaying image data outputted by the signal processor 19. Typically, a liquid crystal display or the like is used as the display apparatus 20. Moreover, the display apparatus 20 may be provided separate from the photoacoustic image-forming apparatus according to the present invention.

### <First example>

An example of a photoacoustic image-forming apparatus to which the present invention is applied will now be described in detail. First, an overall configuration will be described using a schematic apparatus diagram in FIG. 2.

In the present example, a YAG laser-excited Ti:Sa laser system that generates second-order harmonics was used as the light source 11. The laser system is capable of irradiating light with a wavelength ranging from 700 to 900 nm on an object. Pulsed light 12 emitted by the laser was set so as to be expanded using the optical system 13 constituted by a mirror, a beam expander, and the like to a radius of around 3 cm and subsequently irradiated on the object 15.

A two-dimensionally arranged piezo probe having 15 x 23 elements (345 elements) was used as the probe 17. The probe 17 is brought into contact with the holding member 21b to receive acoustic waves. Therefore, a selected area system is adopted which is only capable of receiving photoacoustic waves that have propagated in a particular direction. The data acquisition system 18 has a function of simultaneously receiving all 345 channels of data from the probe, amplifying and digitally converting analog data, and transmitting the converted data to a PC that is the signal processor 19.

A phantom that simulates an object and is made by solidifying 1% intralipid and diluted ink using agar was used as the object 15. In addition, a spherical body that is 2 mm in diameter and colored by black ink was embedded in the phantom as the light absorber 14.

The holding member 21a on the opposite side of the probe 17 (light-irradiating side) is a convex region where a segment that comes into contact with the object protrudes toward the object. The holding member 21a is made of acryl. On the other hand, the holding member 21b on the side of the probe has a flat plate shape and is made of polymethylpentene. The holding members are both 20 x 15 cm. A maximum thickness of the convex shape is 30 mm and a thickness of the flat plate section is 10 mm. Moreover, a curvature radius of the convex shape is 40 cm. The object 15 was lightly compressed and held by the two holding members 21 configured as described above.

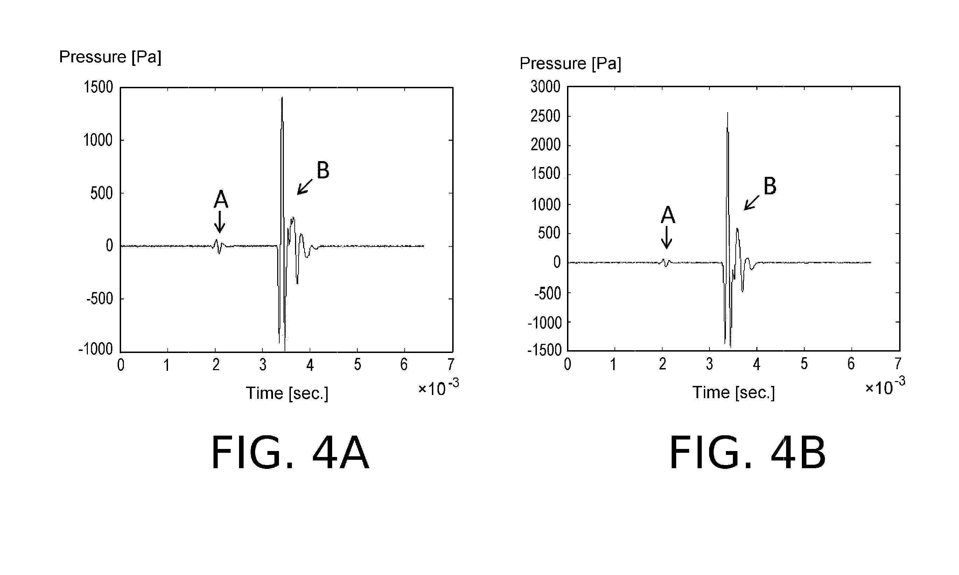

Subsequently, the probe 17 was brought into contact with the planar holding member 21 and light with a wavelength of 800 nm was irradiated on the phantom in a direction opposite to the probe as shown in FIG. 2. A received signal obtained at this point was amplified by an amplifier in the data acquisition system 18, converted from an analog signal to a digital signal, and saved in a PC that is the signal processor 19. An example of the signal obtained at this point is shown in FIG. 4A.

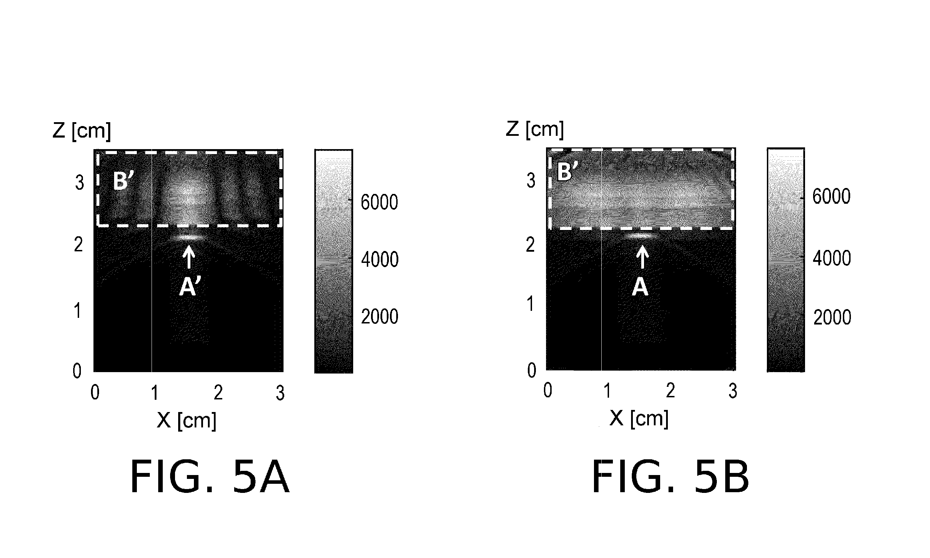

Subsequently, the PC that is the signal processor 19 calculated image data of an initial sound pressure distribution that is an optical constant value distribution in the phantom through image reconstruction. In this case, back projection was used as the image reconstruction method to calculate initial sound pressure distribution data. An example of a reconstructed image obtained at this point is shown in FIG. 5A.

Next, for comparison, the holding member 21a on the opposite side of the probe 17 was changed to a same flat plate shape as the probe-side holding member 21b and a signal was acquired by a similar method. In other words, a measurement was performed using a holding member with a flat plate shape similar to that of the conventional holding member shown in FIG. 1. An example of the signal obtained in this case is shown in FIG. 4B. In addition, the received signal was used to calculate initial sound pressure distribution data through image reconstruction in a similar manner. An example of the reconstructed image obtained in this case is shown in FIG. 5B.

Here, FIG. 4A is compared with FIG. 4B. In FIGS. 4A and 4B, abscissas represent sampling time, whereby the moment of light irradiation is set to 0 seconds. Ordinates represent received sound pressure. Note that scales of the ordinates differ from one another. In addition, in both diagrams, a signal in a region indicated by an arrow A is a photoacoustic signal from the 2 mm-diameter spherical absorber inside the phantom. Meanwhile, a signal in a region indicated by an arrow B is a received signal of photoacoustic waves generated from a light irradiating-side surface of the phantom that is opposite to the probe.

FIG. 4A is compared with FIG. 4B while taking the scales of the ordinates into consideration. Signals in the region A are similar in reception intensity and shape. On the other hand, signals in the region B significantly differ from one another in shapes and peak intensities of the signals. In particular, the peak intensity of the region B in FIG. 4A is approximately half of the peak intensity of the region B in FIG. 4B. This demonstrates that configuring the light irradiating-side holding member 21a so that the region that comes into contact with the object has a convex shape enables a significant reduction in a maximum reception intensity of photoacoustic waves generated from the light irradiating-side object surface.

Next, FIG. 5A is compared with FIG. 5B. FIG. 5 shows maximum intensity projection (MIP) images that are projections of maximum brightness of three-dimensional initial sound pressure distribution data in a direction where 23 probe elements are arranged (Y direction).

In FIGS. 5A and 5B, regions with high brightness (white regions) represent regions with high initial sound pressure and regions with low brightness (black regions) represent regions with low initial sound pressure. In addition, an oval high-brightness region indicated by an arrow A' is a region representing the 2 mm-diameter spherical light absorber inside the phantom, while a region enclosed by a dashed line B' is an artifact (unwanted image) caused by photoacoustic waves generated from the phantom surface.

As is apparent from FIGS. 5A and 5B, although the oval image of the region A' remains unchanged between FIGS. 5A and 5B, the artifact of the region B' is obviously smaller in FIG. 5A than in FIG. 5B. This shows that configuring the holding member 21 so that the region that comes into contact with the object has a curved shape enables an artifact caused by photoacoustic waves generated from the light-irradiating side object surface to be reduced.

### <Second example>

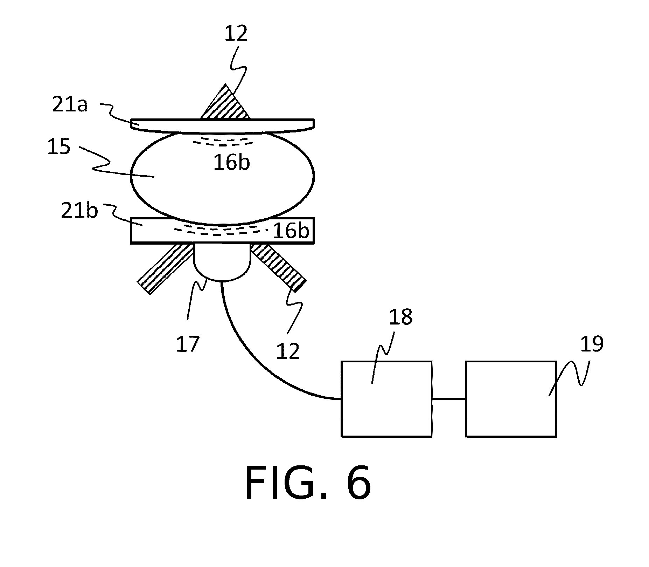

As a second example, an example of a photoacoustic image-forming apparatus in which the two holding members 21 are respectively given a convex shape and a concave shape will be described with reference to FIG. 6. A basic configuration of the apparatus according to the present example is similar to that according to the first example.

However, in the present example, light 12 outputted from the light source 11 (not shown) is guided to each of the two holding members and irradiated on the object 15 from both sides.

In addition, in the present example, an object-contacting surface of the holding member 21b arranged between the probe 17 and the object 15 has a concave shape (a shape that is depressed with respect to the object). As the object 15, a phantom that simulates an object is used in a similar manner to the first example.

After lightly compressing and holding the object 15 by the two holding members 21 configured as described above, light with a wavelength of 756 nm was irradiated from both sides of the phantom as shown in FIG. 6. Moreover, unlike the first example, the light 12 was also irradiated from a probe direction. In a similar manner to the first example, a signal was received and an initial sound pressure distribution inside the phantom was calculated using the received signal. Furthermore, in a similar manner to the first example, a comparison was made with a case where the phantom is held by two conventional holding members 21 having flat plate shapes.

As a result, reception intensity of photoacoustic waves generated from the phantom surface was reduced to equal to or less than half, and an artifact in the image caused by the signal was significantly reduced. As shown, by configuring the region of the holding member 21 that comes into contact with the object in a shape that prevents directions of tangential planes of the region from becoming constant, an artifact in a reconstructed image can be reduced.

### <Third example>

As a third example, an example of a photoacoustic image-forming apparatus in which a shape of the holding member 21 differs from those of the first and second examples will be described. A basic configuration of the apparatus according to the present example is similar to that according to the second example. In other words, light from the light source 11 is irradiated on an object from both sides of the two holding members.

In the present example, a holding member 21b with an object-contacting surface having a wave shape such as that shown in FIG. 3B was used between the probe 17 and the object 15. In addition, an object-contacting surface of the holding member 21a on an opposite side to the probe 17 was given a sawtooth shape as shown in FIG. 3C. The object 15 is a phantom that simulates an object in a similar manner to the examples described earlier.

After lightly compressing and holding the object 15 by the two holding members 21 configured as described above, light with a wavelength of 756 nm was irradiated from both sides of the phantom as shown in FIG. 6. In addition, in a similar manner to the first example, a signal was received and an initial sound pressure distribution inside the phantom was calculated using the received signal. Furthermore, in a similar manner to the first example, a comparison was made with a case where the phantom is held by two conventional holding members 21 having flat plate shapes. As a result, reception intensity of photoacoustic waves generated from the phantom surface was reduced to equal to or less than half, and an artifact in the image caused by the signal was significantly reduced.

As shown, by configuring regions of the holding members 21 that come into contact with the object in a shape that prevents directions of tangential planes of holding sections from becoming constant, an artifact in a reconstructed image can be reduced.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.
An object information acquiring apparatus according to the present invention has: a light source emitting light; a holding member holding an object; a probe receiving an acoustic wave, generated by the object when the light is irradiated on the object via the holding member, and outputting a received signal; and a signal processor acquiring information extracted from the object by using the received signal, wherein a region of the holding member that comes into contact with the object is configured as a non-uniform region that is a region where a normal direction of a tangential plane between the holding member and the object is not constant.

## Claims

1. An object information acquiring apparatus comprising:
a light source configured to emit light;
a holding member configured to hold an object;
a probe configured to receive an acoustic wave, generated by the object when the light is irradiated on the object via the holding member, and output a received signal; and
a signal processor configured to acquire information from the object by using the received signal, wherein
a region of the holding member that comes into contact with the object is configured as a non-uniform region that is a region where a normal direction of a tangential plane between the holding member and the object is not constant.

2. The object information acquiring apparatus according to claim 1, wherein
the holding member is made up of two members that compress and hold the object,
the light emitted by the light source is irradiated on the object via at least one of the holding members,
the non-uniform region is provided on one of the holding members, and
the probe is contact with the holding member on an opposite side to the one holding member and receives the acoustic wave via the opposite-side holding member.

3. The object information acquiring apparatus according to claim 2, wherein
the non-uniform region has a convex shape that protrudes toward the object.

4. The object information acquiring apparatus according to claim 2, wherein
the non-uniform region has a concave shape that is depressed with respect to the object.

5. The object information acquiring apparatus according to claim 2, wherein
the non-uniform region has a wave shape.

6. The object information acquiring apparatus according to claim 2, wherein
the non-uniform region has a sawtooth shape.

7. The object information acquiring apparatus according to any one of claims 2 to 6, wherein
the light emitted by the light source is also irradiated on the object via the holding member from the side of the holding member that comes into contact with the probe, and
an object-contacting region of the holding member that comes into contact with the probe is configured as a non-uniform region.
